# EUROPÄISCHE PATENTSCHRIFT

(11) **EP 2 407 132 B1**
(45) Veröffentlichungstag und Bekanntmachung des Hinweises auf die Patenterteilung: **30.04.2014**
(21) Anmeldenummer: 10006607.5
(22) Anmeldetag: 24.06.2010
(51) Int. Cl.: A61F 9/01, A61F 9/008

(54) **Vorrichtung zum Präparieren eines Auges für das Einbringen von Photosensibilisator**
Device for preparing an eye for introducing a photostabilizer
Dispositif de préparation d'un oeil pour l'introduction d'un photo-stabilisateur

(43) Veröffentlichungstag der Anmeldung: 18.01.2012
(73) Patentinhaber: WaveLight GmbH, 91058 Erlangen (DE)
(72) Erfinder: Seiler, Theo, Prof.Dr. Dr., 8002 Zürich (CH); Seiler, Theo G., jun., 8002 Zürich (CH); Krause, Johannes, 90409 Nürnberg (DE)
(74) Vertreter: von Hellfeld, Axel

(56) Entgegenhaltungen:
- EP-A1- 1 854 438
- EP-B1- 1 561 440
- US-A1- 2009 187 171

## Beschreibung

Die Erfindung betrifft eine Vorrichtung zum Präparieren eines Auges für das Einbringen von Photosensibilisator in Augengewebe mit einer Quelle für Laserstrahlung, Mitteln zum Führen und Fokussieren der Laserstrahlung in Bezug auf das Augengewebe und mit einem Rechner zum Steuern der genannten Mittel. Auch betrifft die Erfindung ein entsprechendes Verfahren zum Präparieren eines Auges für das Einbringen von Photosensibilisator unter Verwendung von Laserstrahlung.

In der Ophthalmologie ist seit mehr als 10 Jahren bekannt, mittels eines sogenannten Photosensibilisators und elektromagnetischer Strahlung die biomechanischen und biochemischen Eigenschaften von Augengewebe, insbesondere der Kornea, zu therapeutischen Zwecken zu ändern.

Der menschliche Augapfel wird durch die äußere Augenhaut begrenzt. Durch den Augeninnendruck wird die kollagenhaltige äußere Augenhaut gespannt und verleiht dem gesunden Augapfel eine annähernd sphärische Form. Im hinteren Augapfelbereich besteht die äußere Augenhaut aus der weißen Lederhaut (Sklera). Im vorderen Bereich befindet sich die für sichtbares Licht durchlässige Hornhaut (Kornea).

Verformungen der äußeren Augenhaut können Ursache einer Fehlsichtigkeit sein. Beispielsweise kann eine Form der Kurzsichtigkeit, die Achsenmyopie, Folge eines skleraren Längenwachstums des Augapfels sein. Eine als Ellipsoid geformte Oberfläche der Kornea kann zu einer Form des Astigmatismus führen, welcher auch als Hornhautverkrümmung bezeichnet wird. Eine weitere Erkrankung der Hornhaut wird als Keratokonus bezeichnet. Beim Keratokonus kommt es infolge einer krankhaften Erweichung der Hornhaut zu einer fortschreitenden Ausdünnung und kegelförmigen Verformung der Hornhaut des Auges. Mit der zunehmenden Auswölbung wird die Hornhaut unterhalb des Zentrums dünner. Sie kann durchbrechen und vernarben. Das setzt die Sehschärfe dauerhaft herab. Die Ursachen des Keratokonus sind heutzutage noch weitgehend unbekannt. Er tritt familiär gehäuft auf, was unter anderem auf eine genetische Disposition schließen lässt. Einen weiteren Risikofaktor für die Entstehung eines Keratokonus stellen Atopien, wie allergische Erkrankungen, dar.

Die konventionelle Therapie eines fortgeschrittenen Keratokonus sieht vor, die erkrankte Kornea zu entfernen und durch ein allogenes Transplantat zu ersetzen. Eine solche Operation ist jedoch eine Organverpflanzung mit den damit verbundenen Risiken und Komplikationen. Ein angemessenes Sehvermögen wird häufig erst ca. zwei Jahre nach der Operation erreicht. Zudem betrifft die Hornhautverpflanzung beim Keratokonus meist junge Menschen, weshalb das Transplantat über Jahrzehnte hinweg einwandfrei funktionieren muss.

Eine demgegenüber verbesserte Therapie des Keratokonus stabilisiert die Kornea durch Vernetzung. Die Behandlung erlaubt eine photochemische, nichtgewebeabtragende Stabilisierung oder Veränderung der biomechanischen und biochemischen Eigenschaften der Kornea. Das Behandlungsprinzip ist auch auf andere betroffene Regionen des Auges anwendbar. Eine Photosensibilisatorlösung wird in das zu verändernde Augengewebe eingebracht und einer Primärstrahlung ausgesetzt. Als Primärstrahlung wird elektromagnetische Strahlung im Wellenlängenbereich von etwa 300 nm bis 800 nm (UV-A-Strahlung oder sichtbares Licht) eingesetzt.

Entsprechende Vorrichtungen zur Behandlung der äußeren Augenhaut sind aus den Druckschriften WO 2007/128581 A2 und WO 2008/000478 A1 bekannt.

Die EP 1 561 440 B1 beschreibt eine Vorrichtung, bei der mit einem relativ komplexen Aufbau eine homogene Verteilung der Strahlung im okulärem Gewebe erzeugt wird. Ein Formkörper wird dort auf die Hornhaut aufgesetzt, um diese in eine gewünschte Form zu bringen während mittels elektromagnetischer Strahlung und des Photosensibilisators das okuläre Gewebe hinsichtlich seiner Festigkeit geändert wird. Ein solcher Formkörper kann auch im Zusammenhang mit der vorliegenden Erfindung eingesetzt werden.

Eine Vorrichtung gemäß WO 2007/128581 A2 dient der Verfestigung der sich im hinteren Augenabschnitt befindenden Sklera. Die Primärstrahlung kann dabei durch das Innere des Auges oder durch von außen aufliegenden Kissen auf die Sklera einwirken. Durch einen Photomediator oder Photosensibilisator wird eine Vernetzung in der Sklera bewirkt. Dadurch wird einem Sklerawachstum entgegengewirkt und ein Fortschreiten der Achsenmyopie unterbunden.

Die EP 1 854 438 A1 beschreibt eine ophthalmalogische Vorrichtung zur Vorbeugung einer Myopie, bei der die Sklera mittels eines Photosensibilisators verfestigt wird.

Die Druckschrift WO 2008/000478 A1 beschreibt ein Bestrahlungssystem zur biomechanischen Stabilisierung der Kornea. Auch hier kann in Verbindung mit einem Photosensibilisator eine Vernetzung an der Kornea bewirkt werden. Das Bestrahlungssystem bietet die Möglichkeit spezifische Erkrankungen, wie den Keratokonus zu therapieren.

Die US 2009/187171 A1 beschreibt das Erzeugen von Schnitten in Volumina innerhalb des Stromas, wobei die Volumina vollständig im Stroma liegen. Damit soll aufgrund des intraokularen Druckes die Form der Kornea geändert werden.

Die Änderung der Gestalt und/oder von mechanischen Eigenschaften von Augengewebe, insbesondere der Hornhaut und allgemein der Sklera mittels eines eingebrachten Photosensibilisators und elektromagnetischer Strahlung ist als solche im Stand der Technik, insbesondere wie oben genannt, gut bekannt. Hinsichtlich der chemischen Zusammensetzung des Photosensibilisators wird auf den Stand der Technik verwiesen, auch hinsichtlich der eingesetzten Art der elektromagnetischen Strahlung, insbesondere der geeigneten Wellenlängen in Verbindung mit bestimmten Photosensibilisatoren.

Einem routinemäßigen Einsatz der Vemetzungstherapie am Augengewebe stehen jedoch komplexe Abhängigkeiten entgegen. Die Beziehungen zwischen den eingesetzten Dosen und deren Wirkung im Augengewebe sind sehr vielfältig. Als Dosis in diesem Sinne kommen insbesondere in Betracht die elektromagnetische Strahlung hinsichtlich ihrer Intensität sowie ihrer Verteilung in Raum und Zeit; der eingesetzte Photosensibilisator hinsichtlich seiner chemischen Struktur, Konzentration, und Einwirkung in Raum und Zeit. Die Wirkungen von unterschiedlichen Dosen dieser Parameter auf und in dem Augengewebe eines Patienten sind sehr stark abhängig von Eigenschaften (Messdaten) bezüglich des Patienten. Dabei ist insbesondere zu berücksichtigen, dass die Wirkung der mit der Strahlung und dem Photosensibilisator durchgeführten Vernetzung auch unerwünscht sein kann bis hin zu einer Schädigung des Augengewebes oder der Funktion des Auges.

Als Photosensibilisator hat sich zur Zeit weitgehend Riboflavin als vorteilhaft erwiesen. Um Riboflavin in die Kornea (Hornhaut) einzubringen, muss im Stand der Technik das Hornhautepithel zumindest teilweise entfernt werden, da es das Eindringen von Riboflavin in die Hornhaut behindert, also sozusagen eine Schranke für die Diffusion der Riboflavin-Moleküle in das Hornhautgewebe darstellt. Allerdings ist das Entfernen des Epithels in der Regel mit Schmerzen für den Patienten verbunden und auch der anschließende Heilungsprozess nicht immer ohne Komplikationen.

Der Erfindung liegt die Aufgabe zugrunde, eine Vorrichtung der eingangs genannten Art bereitzustellen, die eine schonende Einbringung des Photosensibilisators in das Augengewebe ermöglicht, insbesondere hinsichtlich der Tiefe. Insbesondere soll auch in einfacher Weise die Quervernetzung im Augengewebe steuerbar sein.

Hierzu stellt die Erfindung eine Vorrichtung gemäß Anspruch 1 bereit zum Präparieren eines Auges für das Einbringen von Photosensibilisator in Augengewebe.

Damit ist es möglich, den Photosensibilisator in einen oder mehrere Kanäle in einfacher Weise einzubringen, ohne dass hierfür erhebliche Teile des Epithels entfernt oder aufgeklappt werden müssen.

Die für die Erzeugung des Kanals oder der Kanäle eingesetzte Laserstrahlung kann mit als solches bekannten Mitteln erzeugt werden. Bekannt sind die für das Erzeugen der Kanäle erforderlichen Quellen für Laserstrahlung zum Beispiel aus der sogenannten Femto-LASIK. Dort wird Laserstrahlung zum Beispiel eines Femtosekundenlasers als sogenanntes "Laserskalpell" eingesetzt, um Augengewebe durch "Verdampfung" (Kavitationsblasen) mit der Energie des Laserlichts zu schneiden. In der Femto-LASIK wird mit dem Laserskalpell der sogenannte Flapschnitt erzeugt, also ein Deckelchen von der Seite her aus dem Epithelium ausgeschnitten und weggeklappt, um dann mit z. B. einem Excimerlaser im freiliegenden Stroma zur Neuformung der Kornea eine Ablation durchzuführen. Auch gepulste Laser mit Pulslängen im Picosekundenbereich und im Nanosekundenbereich sind zur Erzeugung der Kanäle geeignet.

Der Begriff "Kanal" im Sinne der vorliegenden Erfindung bedeutet keine Schnittfläche zur Erzeugung eines sogenannten Flaps, wie er bei der Femtosekunden-LASIK bekannt ist.

Die für die vorliegende Erfindung eingesetzten Mittel zum Führen und Fokussieren der Laserstrahlung in Bezug auf das Augengewebe können dieser Technik entnommen werden. Gemäß der Erfindung wird der die optischen Mittel zum Führen und Fokussieren der Laserstrahlung steuernde Rechner aber erfindungsgemäß derart programmiert, dass die Fokusse der Laserstrahlung nacheinander so entlang einer geraden oder gekrümmten Linie bewegt werden, dass durch sogenannte Kavitationsblasen im Gewebe ein Kanal oder mehrere Kanäle entstehen, die ausgehend von der Oberfläche des Augengewebes, also insbesondere der Hornhaut, in das Innere des Augengewebes reichen, so dass ein Photosensibilisator, der an den Eingang des Kanals gebracht wird, in den Kanal und damit in das Innere des Augengewebes eindringen kann. Dabei werden die Kanäle bevorzugt so erzeugt, dass die einzelnen benachbarten Kavitationsblasen jeweils einen solchen Abstand zueinander haben ("spacing"), dass die Struktur und Stabilität des Gewebes möglichst wenig beeinträchtigt wird. Der Abstand zwischen den Kavitationsblasen, welche den Kanal erzeugen, soll aber auch andererseits so gering sein, dass der in Form einer Lösung in den Kanal eingeführte Photosensibilisator, z. B. Riboflavin, in der gewünschten Weise durch den Kanal, also sozusagen von Kavitationsblase zu Kavitationsblase in das Gewebe eindringt. In den Bereichen zwischen benachbarten Kavitationsblasen erfolgt also das Eindringen der Photosensibilisator-Lösung durch Diffusion. Somit ist im Sinne dieser Erfindung der Begriff "Kanal" nicht notwendig als durchgehender, völlig von Gewebe befreiter Hohlraum zu verstehen, obwohl andererseits auch völlig durchgehende Kanäle im Sinne der Erfindung denkbar sind.

Gemäß einer Variante der Erfindung kann der Kanal auch einer gekrümmten Linie folgen, da durch die Fokussierung der Laserstrahlung im Inneren des Gewebes mit Fokus-Punkten entlang der gekrümmten Linie auch eine von der geraden Linie abweichende Kanalform erzeugbar ist. Bei allen genannten Kanalformen kann durch Aneinanderreihung der Fokusse der Laserstrahlung mit hinreichend dichtem Abstand durch sie sogenannte "Photodisruption" der Kanal mit gewünschtem Durchmesser und mit gewünschtem geometrischen Verlauf erzeugt werden.

Die Erfindung ermöglicht es auch, die Dichte der Kanäle im Augengewebe unterschiedlich, abhängig vom Ort im Augengewebe, einzustellen, also an gewünschten Stellen des Augengewebes mehr Kanäle zu setzen als an anderen Stellen, was dann zur Folge hat, dass dort, wo die Kanaldichte größer ist, auch die Dichte des Photosensibilisators im Gewebe höher ist und damit auch die biomechanische und biochemische Wirkung an solchen Stellen anders ist als an jenen Stellen im Augengewebe, an denen die Kanaldichte geringer ist.

Auch kann die Dichte des im Augengewebe letztlich zur Wirkung kommenden Photosensibilisators dadurch gesteuert werden, dass die Tiefe der Kanäle in der Kornea ortsabhängig variiert wird.

Ebenso kann die Dichte des in das Augengewebe einzubringenden Photosensibilisators dadurch gesteuert werden, dass der Querschnitt des einen oder der mehreren Kanäle mehr oder weniger groß gewählt wird.

Die Breite eines Kanals liegt bevorzugt im Bereich von 0,1 mm² bis 1,2 mm, wobei jedes Subintervall darin ebenfalls hier offenbart ist.

Wenn hier von "Kanal" die Rede ist, meint dies immer den Singular oder den Plural.

Mit der erfindungsgemäßen Vorrichtung kann also ein Kanalsystem in der Hornhaut erzeugt werden, das einen Zugang von außen in das Innere der Hornhaut ermöglicht. Die Photosensibilisator-Lösung kann dann eingespritzt werden, so dass sie sich im Hornhautstroma verteilt. Hierfür kann das Kanalsystem, je nach der ophthalmologischen Indikation, einen oder mehrere Kanäle aufweisen, wobei zum Beispiel bei einer gewünschten homogenen Verteilung des Photosensibilisators die Dichte der Kanäle (also die Anzahl der Kanäle pro Flächeneinheit oder pro Volumeneinheit) im Wesentlichen homogen ist im behandelten Bereich der Kornea. Zum Beispiel können vier Kanäle in die vier Korneasegmente gesetzt werden, entsprechend den vier Segmenten der Projektion der Kornea auf eine Ebene. Das Kanalsystem kann auch stochastisch generiert werden.

Auch können die Querschnitte der einzelnen Kanäle durch entsprechende Steuerung der Fokusse der Laserstrahlung wahlweise gestaltet werden, zum Beispiel kreisförmig, rechteckförmig, quadratisch, oder auch oval bzw. schlitzförmig.

Bevorzugt erstrecken sich die Kanäle im Wesentlichen quer zur Achse (A) des Auges. Der Begriff "quer" bedeutet hier im Wesentlichen "radial". "Radial" bedeutet eine Richtung ausgehend vom Apex der Kornea nach außen.

Eine Ausgestaltung der Erfindung sieht vor, dass der oder die Kanäle im Wesentlichen die gesamte radiale Fläche der Kornea mit im Wesentlichen gleichförmiger Kanaldichte durchziehen. Dies bedeutet, mit anderen Worten, dass mindestens in einer vorgegebenen Fläche in vorgegebener Tiefe der Kornea Photosensibilisator durch Diffusion homogen (gleichförmig mit gleicher Dichte) in das Komeagewebe gebracht wird.

Es ist vorgesehen, dass der eine oder die mehreren Kanäle mit mehr als einer Öffnung verbunden sind, wobei die genannte

Öffnung in die Oberfläche des Augengewebes reicht, so dass Photosensibilisator ohne Hindernis in den oder die Kanäle einbringbar ist, z. B. mit einer feinen Spritze oder dergleichen.

Die genannten Öffnungen, durch welche die Kanäle von außen zugänglich sind, werden bevorzugt an oder nahe dem Rand der Kornea, also an oder nahe dem Limbus, angeordnet.

Die Erzeugung von Kanälen oder Hohlräumen im Sinne der vorliegenden Erfindung ist also verschieden von der Erzeugung eines sogenannten Flaps für die LASIK, d. h. die erfindungsgemäßen Kanäle bzw. Hohlräume bewirken nicht die Erzeugung eines Flaps ("Deckelchens"), welches zur Seite geklappt werden kann.

Eine andere bevorzugte Ausgestaltung der Erfindung sieht vor, dass zumindest ein Teil der Kanäle spiralförmig verläuft.

Bei allen beschriebenen Ausgestaltungen und Ausführungsbeispielen der Erfindung kann auch ein Gas, insbesondere Luft, in einen oder mehrere Kanäle bzw. Hohlräume injiziert werden.

Weitere bevorzugte Ausgestaltungen der Erfindung sind in den weiteren abhängigen Ansprüchen beschrieben.

Nachfolgend wird die Erfindung mit weiteren Einzelheiten anhand der Zeichnung näher erläutert.

Es zeigt:
Fig. 1 schematisch eine Vorrichtung zum Präparieren eines Auges für das Einbringen eines Photosensibilisators in Augengewebe;
Fig. 2 eine Hornhaut in Draufsicht mit einer schematischen Erläuterung der Erzeugung von Kanälen darin;
Fig. 3 ein anderes Ausführungsbeispiel der Erfindung, bei der der Rechner so programmiert ist, dass er etwa kreissektorförmige Kanalgebilde im Augengewebe erzeugt zur Behandlung von insbesondere Astigmatismus;
Fig. 4 einen axialen Schnitt durch eine Kornea mit Kanälen, die in unterschiedlichen Tiefen, bezogen auf die Oberfläche der Kornea, verlaufen; und
Fig. 5 eine axiale Draufsicht auf eine Kornea, bei der zumindest ein Ring als Kanal geformt ist zur Behandlung von Hyperopie.

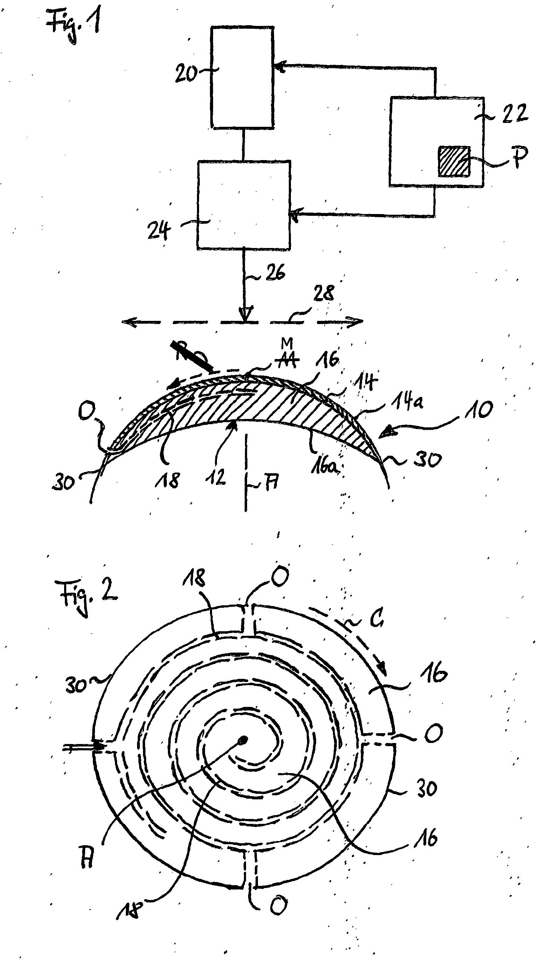

Fig. 1 zeigt schematisch ein Auge 10, das durch Einbringen von Photosensibilisator hinsichtlich seiner biomechanischen und/oder biochemischen Eigenschaften verändert werden soll. Als solches ist dieser Vorgang als "korneales Crosslinking" bekannt. Zum Beispiel kann durch das "Crosslinking", also die sogenannte Quervernetzung, die mechanische Stabilität der Hornhaut verstärkt werden. Durch Einsatz von Formkörpern kann auch die Form der Hornhaut während der Kanalentstehung oder der Quervernetzung verändert werden. Darüber hinaus ist der Einsatz von Photosensibilisatoren auch geeignet, infektiöse Entzündungen der Hornhaut zu behandeln, wobei die entstehenden Radikale eingedrungene Keime töten.

Eine Augenachse ist mit "A" bezeichnet, wobei diese Augenachse im wesentlichen auch zusammenfällt mit der optischen Achse der weiter unten näher beschriebenen Mittel zum Führen und Fokussieren von Laserstrahlung.

Das Zentrum (Mittelpunkt) der Hornhautoberfläche (14a) ist mit "M" bezeichnet, so dass hiervon ausgehend eine radiale Richtung R definiert werden kann.

Das durch Quervernetzung zu behandelnde Augengewebe 12 ist hier im wesentlichen die Hornhaut (Kornea) 16, die nach außen durch das Epithel 14 abgedeckt ist.

Mit der noch näher zu beschreibenden Vorrichtung werden Kanäle 18 in das Stroma der Hornhaut 16 eingebracht, wobei die Kanäle 18 mit Öffnungen 0 (Ports) flüssigkeitsleitend verbunden sind und die Öffnungen O einen Zugang von außen in die Kanäle für das Eingeben von Photosensibilisator-Lösung ermöglichen. Die Kanäle 18 reichen in das Innere der Hornhaut 16 und enden vor deren innerer Oberfläche 16a.

In die Kanäle 18 wird ein Photosensibilisator, z.B.Riboflavin, eingebracht, welches in die Kanäle eindringt und sich von dort ausgehend durch Diffusion im Hornhautgewebe verteilt.

Die Vorrichtung weist eine Quelle 20 für Laserstrahlung auf, z.B. einen oben erläuterten Femtosekundenlaser, wie er z.B. zum Schneiden eines Flap in der Femto-LASIK eingesetzt wird. Auch hinsichtlich der Mittel 24 zum Führen und Fokussieren der Laserstrahlung 26 im Inneren der Hornhaut 16 kann auf entsprechende Mittel, wie sie aus der Femto-LASIK als solches bekannt sind, zurückgegriffen werden.

Im Unterschied zur LASIK ist ein Rechner 22, der die Laserstrahlquelle 20 und die optischen Mittel 24 zum Führen und Fokussieren der Laserstrahlung 26 steuert, mit einem Programm P programmiert, das die Laserstrahlung 26 in besonderer Weise zur Erzeugung der Kanäle 18 steuert. Hierzu wird bei Erzeugung der genannten Kanäle 18 gemäß Fig. 1 die Laserstrahlung 26 in Richtung der Pfeile 28 parallel verschoben. Die Darstellung gemäß Fig. 1 zeigt einen die Achse A enthaltenden Schnitt durch das Auge. In Fig. 1 ist ein Kanal 18 gezeigt, der im Wesentlichen parallel zur Oberfläche 14a der Kornea verläuft. Der Kanal ist über eine Öffnung O, die nahe dem Limbus 30 liegt, von außen zugänglich. Zum Beispiel kann eine feine Spritze in die Öffnung O eingeführt werden, um eine Photosensibilisator-Lösung oder ein Gas, wie Luft, in den Kanal 18 einzuspritzen.

Fig. 2 zeigt eine Draufsicht auf eine Kornea 16 und einen im Inneren der Kornea 16 verlaufenden Kanal 18, der beim dargestellten Ausführungsbeispiel spiralförmig ist mit zusätzlichen Öffnungen O, die in Umfangsrichtung C unter Abständen verteilt sind. Die etwa spiralförmig verlaufenden Kanäle 18 sind beim dargestellten Ausführungsbeispiel in einer Fläche angeordnet, die sich im Wesentlichen parallel zur Oberfläche 14a des Auges erstreckt. In Abwandlung dieses Ausführungsbeispieles können die Kanäle 18 auch in einer Ebene angeordnet sein, die senkrecht zur Achse A steht. Wiederum eine Abwandlung sieht vor, dass die Kanäle in einer Fläche verlaufen, die sich parallel zur Rückfläche 16a der Kornea 16 erstreckt. Die Wahl des Ortes und des Verlaufs der Kanäle 18 kann von der gegebenen medizinischen Indikation abhängen und entsprechend gewählt werden.

Beim dargestellten Ausführungsbeispiel gemäß Fig. 2 sind die Kanäle so positioniert, dass sie zumindest in der von ihnen aufgespannten Fläche bewirken, dass sich der Photosensibilisator dort durch Diffusion im Wesentlichen homogen im Korneagewebe verteilt.

In Abwandlung der in den Figuren dargestellten Ausführungsbeispiele können Kanäle auch axial, d.h. parallel zur Achse A verlaufen, zumindest teilweise.

Kanäle können sich auch radial erstrecken.

Auch sind alle vorstehend genannten Verläufe und Anordnungen der Kanäle wahlweise miteinander kombinierbar.

Durch die Wahl der Durchmesser und geometrischen Anordnung der Kanäle kann auch die Verteilung von Photosensibilisator in der Kornea wahlweise, je nach medizinischer Indikation, gesteuert werden.

Die Kanäle werden durch fokussierte Laserstrahlung, insbesondere mittels eines Femtosekundenlasers, durch Kavitationsblasen, die von den Laserfokussen erzeugt werden, gebildet. Dabei ist bevorzugt vorgesehen, dass benachbarte Kavitationsblasen sich nicht vollständig überlagern, so dass zwischen einzelnen Kavitationsblasen Gewebereste verbleiben, die einerseits das Gesamtgewebe in der Struktur stabilisieren und andererseits hinreichend durchlässig sind für die Diffusion von Photosensibilisator in den Kanälen.

Es ist auch möglich, statt langgestreckter Kanäle anders geformte Hohlräume durch die genannten Kavitationsblasen zu erzeugen, insbesondere flächige Hohlräume, in denen z. B. unter regelmäßigen kürzeren Abständen Gewebebereiche als "Pfosten" zwischen den Ober- und Unterflächen des Hohlraumes oder der Hohlräume verbleiben.

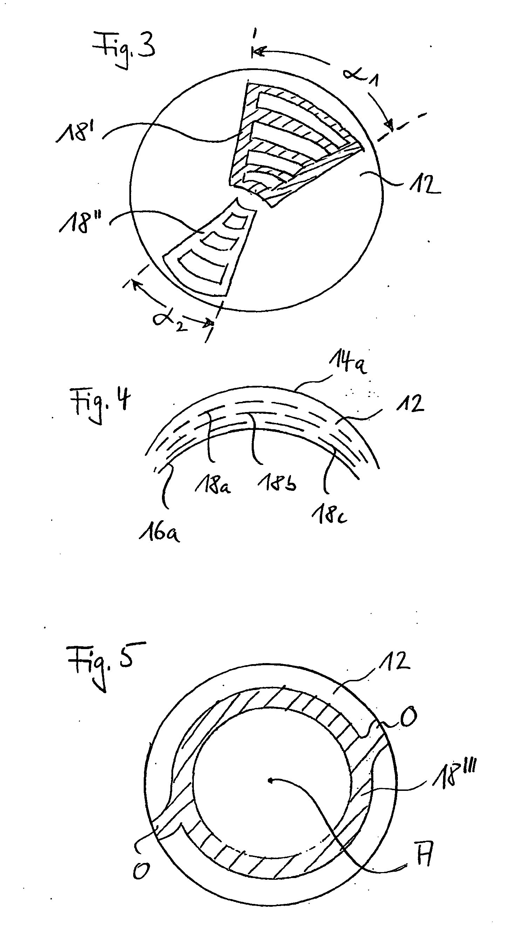

Fig. 3 zeigt in axialer Draufsicht eine Kornea mit einem Kanalsystem 18', 18", das zur Behandlung von Astigmatismus in der Kontur etwa kreissektorförmig (wie dargestellt) geformt ist. Dabei können z. B. gemäß Fig. 3 zwei kreissektorförmige Kanalsysteme 18' bzw. 18" ausgeformt sein, mit jeweils einem unterschiedlichen Sektorwinkel α₁ bzw. α₂.

Fig. 4 zeigt Kanäle 18a, 18b, 18c, die in unterschiedlichen Tiefen, bezogen auf die Oberfläche 14a der Kornea, verlaufen. Die in Fig. 4 schematisch gezeigten unterschiedlichen drei Tiefen für die Kanäle können für sämtliche hier im Einzelnen mitgeteilten Strukturen und Anordnungen von Kanälen gemäß den Figuren 1, 2, 3 und 5 sowie anderen Ausführungsbeispielen verwirklicht werden.

Fig. 5 zeigt einen ringförmigen Kanal 18"' in schematischer Draufsicht mit zwei Öffnungen O, die den Kanal 18"' mit der Oberfläche der Kornea verbinden. In Abwandlung des Ausführungsbeispiels gemäß Fig. 5 können auch mehrere ringförmige Kanäle vorgesehen sein, die entweder untereinander und/oder jeweils einzeln mit der Oberfläche der Kornea flüssigkeitsleitend verbunden sind.

Die Erfindung beinhaltet auch ein Verfahren zum Präparieren eines Auges für das Einbringen von Photosensibilisator, wobei mittels Laserstrahlung 26, die auf und in die Hornhaut fokussiert wird, Kanäle 18 in der Hornhaut erzeugt werden, die sich von der Oberfläche 14a der Kornea ausgehend in das Innere der Kornea erstrecken. Bei diesem Verfahren können alle oben beschriebenen Merkmale und Eigenschaften der Kanäle 18 eingesetzt werden.
- 10: Auge
- M: Mittelpunkt (von 10)
- A: Augenachse
- P: Programm
- C: Umfangsrichtung
- O: Öffnung
- 12: Augengewebe
- 14: Epithel, 14a Oberfläche (von 14)
- 16: Kornea, 16a Rückfläche (von 16)
- 18: Kanal
- 20: Quelle für Laserstrahlung
- 22: Rechner
- 24: Mittel
- 26: Laserstrahlung
- 28: Pfeile
- 30: Limbus

## Patentansprüche

1. Vorrichtung zum Präparieren eines Auges (10) für das Einbringen von Photosensibilisator in Augengewebe (12) mit
- einer Quelle (20) für Laserstrahlung,
- Mitteln (24) zum Führen und Fokussieren der Laserstrahlung (26) in Bezug auf das Augengewebe (12), und mit
- einem Rechner (22) zum Steuern der genannten Mittel (24), wobei der Rechner (22) programmiert ist, die Laserstrahlung (26) so zu steuern, dass sie im Augengewebe (12) zumindest einen Kanal (18), der in das Inneren des Augengewebes reicht, und eine oder mehrere Öffnungen (O) in der Oberfläche des Augengewebes (14a) erzeugt,
**dadurch gekennzeichnet, dass** der Rechner programmiert ist, die Mittel (24) so zu steuern, dass der oder die mehreren Kanäle (18) mit einer oder mehreren Öffnungen (O) in der Oberfläche (14a) des Augengewebes verbunden sind.

2. Vorrichtung nach Anspruch 1, **dadurch gekennzeichnet, dass** der Kanal oder die mehreren Kanäle (18) sich im Wesentlichen quer zur Achse (A) des Auges erstrecken.

3. Vorrichtung nach einem der Ansprüche 1 oder 2, **dadurch gekennzeichnet, dass** der Rechner (22) programmiert ist die Mittel (24) so zu steuern, dass sie, einen oder mehrere Kanäle (18) entlang von Strecken in das Augengewebe (12) einbringen, die sich im Wesentlichen radial (R) erstrecken.

4. Vorrichtung nach einem der Ansprüche 1, 2 oder 3, **dadurch gekennzeichnet, dass** der oder die mehreren Kanäle (18) im Wesentlichen die gesamte radiale Fläche der Kornea (16) mit im Wesentlichen gleichförmiger Kanaldichte durchziehen.

5. Vorrichtung nach einem der vorhergehenden Ansprüche, **dadurch gekennzeichnet, dass** sich zumindest ein Teil der Kanäle spiralförmig erstreckt.

6. Vorrichtung nach einem der vorhergehenden Ansprüche, **dadurch gekennzeichnet, dass** der Kanal oder die Kanäle (18) zumindest teilweise durch von der Laserstrahlung erzeugte Kavitationsblasen erzeugt sind, die zumindest teilweise nicht kontinuierlich ineinander übergehen, wobei der Abstand zwischen benachbarten Kavitationsblasen im Bereich von 1 bis 50 µm liegt, bevorzugt im Bereich von 5 bis 30 µm, und besonders bevorzugt im Bereich von 10 bis 20 µm.

7. Vorrichtung nach einem der vorhergehenden Ansprüche, **dadurch gekennzeichnet, dass** zumindest ein Teil der Kanäle sich zumindest teilweise axial und/oder zumindest teilweise gekrümmt erstrecken.

8. Vorrichtung nach Anspruch 1, **dadurch gekennzeichnet, dass** der Rechner (22) programmiert ist die Mittel (24) so zu steuern, dass sie, Kanäle (18) in das Augengewebe (12) einbringen, deren Dichte abhängig vom Ort im Augengewebe variiert.

9. Vorrichtung nach einem der vorhergehenden Ansprüche, **dadurch gekennzeichnet, dass** der Rechner (22) programmiert ist die Mittel (24) so zu steuern, dass sie, Kanäle (18) in das Augengewebe (12) einbringen, deren Tiefe und/oder deren Querschnitt abhängig vom Ort im Augengewebe variiert.

10. Vorrichtung nach einem der vorhergehenden Ansprüche, **dadurch gekennzeichnet, dass** der Rechner (22) programmiert ist die Mittel (24) so zu steuern, dass sie, Kanäle (18) in das Augengewebe (12) einbringen, wobei verschiedene Kanäle unterschiedliche Querschnitte aufweisen.

11. Vorrichtung nach einem der vorhergehenden Ansprüche, **dadurch gekennzeichnet, dass** die Quelle (20) für Laserstrahlung ein Femtosekundenlaser, ein Nanosekundenlaser, oder ein Picosekundenlaser ist.

12. Vorrichtung nach einem der vorhergehenden Ansprüche, **dadurch gekennzeichnet, dass** der Rechner programmiert ist die Mittel (24) so zu steuern, dass sie, zur Therapie von Astigmatismus Kanäle (18', 18") im Augengewebe erzeugen, die etwa die Form von Kreissektoren annehmen.

13. Vorrichtung nach einem der vorhergehenden Ansprüche, **dadurch gekennzeichnet, dass** der Rechner (22) programmiert ist die Mittel (24) so zu steuern, dass sie, einen oder mehrere Kanäle (18'") erzeugen, die zumindest annähernd ringförmig verlaufen.

14. Vorrichtung nach einem der vorhergehenden Ansprüche, **dadurch gekennzeichnet, dass** der Rechner (22) programmiert ist die Mittel (24) so zu steuern, dass sie, Kanäle (18a, 18b, 18c) im Augengewebe (12) erzeugen, die in unterschiedlichen Tiefen im Augengewebe verlaufen.

## Claims

1. An apparatus for preparing an eye (10) for applying a photosensibilisator into the eye's tissue (12), comprising:
- a source (20) for laser radiation,
- means (24) for guiding and focusing the laser radiation (26) in relation to the eye's tissue (12), further comprising
- a computer (22) for controlling said means (24), wherein the computer (22) is programmed to control the laser ration (26) such that the laser radiation generates in the eye's tissue (12) at least one channel (18) extending to the inner of the eye's tissue and generates one or more orifices (O) in the surface (14a) of the eye's tissue,
**characterized in that** the computer is programmed to control said means (24) such that the one or more channels (18) are communicating with one or more orifices (O) in the surface (14a) of the eye's tissue.

2. The apparatus according to claim 1, **characterized in that** the one or more channels (18) extend a substantially transverse to the axis (A) of the eye.

3. The apparatus according to claim 1 or 2, **characterized in that** the computer (22) is programmed to control said means (24) such that the one or more channels (18) are applied into the eye's tissue (12) along lines extending substantially radially (R).

4. The apparatus according to any one of claims 1, 2 or 3, **characterized in that** the one or more channels (18) penetrate substantially the entire radial surface of the cornea (16) while having a substantially uniform channel density.

5. The apparatus according to any one of the preceding claims, **characterized in that** at least a part of the channels extends spirally.

6. The apparatus according to any one of the preceding claims, **characterized in that** the one or more channels (18) are generated at least partially by cavitation bubbles generated by the laser radiation, wherein the cavitation bubbles partially do not transit continually into one another, wherein the distance between neighboring cavitation bubbles is in the range from 1 to 50 µm, preferably in the range from 5 to 30 µm and particularly preferable in the range of 10 to 20 µm.

7. The apparatus according to any one of the preceding claims, **characterized in that** at least a part of the channels extends at least partially axially and/or at least partially curved.

8. The apparatus according to claim 1, **characterized in that** the computer (22) is programmed so as to control said means (24) such that channels (18) are applied into the eye's tissue (12), the density of which varies depending on the site in the eye's tissue.

9. The apparatus according to any one of the preceding claims, **characterized in that** the computer (22) is programmed to control said means (24) such that channels (18) are applied into eye's tissue (12), the depth and/or the section of which varies depending on the site in the eye's tissue.

10. The apparatus according to any one of the preceding claims, **characterized in that** the computer (22) is programmed to control said means (24) such that channels (18) are applied into the eye's tissue (12), wherein different channels have different sections.

11. The apparatus according to any one of the preceding claims, **characterized in that** the source (20) for laser radiation is one of a femtosecond laser, a nanosecond laser or a picosecond laser.

12. The apparatus according to any one of the preceding claims, **characterized in that** the computer is programmed to control said means (24) such that channels (18', 18") are generated into the eye's tissue for therapy of astigmatism, which channels approximately take the form of sectors.

13. The apparatus according to any one of the preceding claims, **characterized in that** the computer (22) is programmed to control said means (24) so as to generate one or more channels (18'") extending at least approximately annularly.

14. The apparatus according to any one of the preceding claims, **characterized in that** the computer (22) is programmed to control said means (24) such that channels (18a, 18b, 18c) are generated into the eye's tissue (12), which channels extend in different depths in the eye's tissue.

## Revendications

1. Dispositif de préparation d'un oeil (10) pour l'introduction d'un photosensibilisateur dans les tissus oculaires (12), comportant
- une source (20) de rayonnement laser,
- des moyens (24) de guidage et de focalisation du rayonnement laser (26) par rapport au tissu oculaire (12), et comportant
- un calculateur (22) pour commander lesdits moyens (24), ledit calculateur (22) étant programmé pour commander le rayonnement laser (26) de telle sorte qu'il forme dans le tissu oculaire au moins un canal (18) qui pénètre l'intérieur du tissu oculaire, ainsi qu'une ou plusieurs ouvertures (O) dans la surface (14a) du tissu oculaire,
**caractérisé en ce que** le calculateur est programmé pour commander les moyens (24) de telle sorte que ledit canal ou lesdits plusieurs canaux (18) sont reliés à une ou à plusieurs ouvertures (O) dans la surface (14a) du tissu oculaire.

2. Dispositif selon la revendication 1, **caractérisé en ce que** le ou les canaux (18) s'étendent pour l'essentiel perpendiculairement à l'axe (A) de l'oeil.

3. Dispositif selon l'une des revendications 1 ou 2, **caractérisé en ce que** le calculateur (22) est programmé pour commander les moyens (24) de telle sorte qu'ils introduisent dans le tissu oculaire (12), le long de voies, un ou plusieurs canaux (18) qui s'étendent pour l'essentiel radialement (R).

4. Dispositif selon l'une des revendications 1, 2 ou 3, **caractérisé en ce que** le ou les canaux (18) traversent pour l'essentiel toute la surface radiale de la cornée (16) avec une densité de canaux sensiblement uniforme.

5. Dispositif selon l'une des revendications précédentes, **caractérisé en ce qu'**au moins une partie des canaux s'étend en spirale.

6. Dispositif selon l'une des revendications précédentes, **caractérisé en ce que** le ou les canaux (18) sont formés au moins pour partie par des bulles de cavitation qui sont produites par le rayonnement laser et ne sont pas, au moins pour partie, reliées de manière continue les unes aux autres, la distance entre des bulles de cavitation voisines étant située dans la plage comprise entre 1 à 50 µm, de préférence dans la plage comprise entre de 5 à 30 µm, et de manière particulièrement préférée dans la plage comprise entre 10 à 20 µm.

7. Dispositif selon l'une des revendications précédentes, **caractérisé en ce qu'**au moins une partie des canaux s'étend au moins pour partie radialement et/ou au moins pour partie de manière incurvée.

8. Dispositif selon la revendication 1, **caractérisé en ce que** le calculateur (22) est programmé pour commander les moyens (24) de telle sorte qu'ils introduisent dans le tissu oculaire (12) des canaux (18) dont la densité varie selon l'endroit où a lieu l'introduction dans le tissu oculaire.

9. Dispositif selon l'une des revendications précédentes, **caractérisé en ce que** le calculateur (22) est programmé pour commander les moyens (24) de telle sorte qu'ils introduisent dans le tissu oculaire (12) des canaux (18) dont la profondeur et/ou la section varient selon l'endroit où a lieu l'introduction dans le tissu oculaire.

10. Dispositif selon l'une des revendications précédentes, **caractérisé en ce que** le calculateur (22) est programmé pour commander les moyens (24) de telle sorte qu'ils introduisent des canaux (18) dans le tissu oculaire (12), les différents canaux présentant des sections différentes.

11. Dispositif selon l'une des revendications précédentes, **caractérisé en ce que** la source (20) de rayonnement laser est constituée par un laser femtoseconde, un laser nanoseconde ou un laser picoseconde.

12. Dispositif selon l'une des revendications précédentes, **caractérisé en ce que** le calculateur (22) est programmé pour commander les moyens (24) de telle sorte qu'ils forment dans le tissu oculaire des canaux (18', 18") qui ont approximativement la forme de secteurs circulaires pour corriger un astigmatisme.

13. Dispositif selon l'une des revendications précédentes, **caractérisé en ce que** le calculateur (22) est programmé pour commander les moyens (24) de telle sorte qu'ils forment un ou plusieurs canaux (18"') qui s'étendent au moins approximativement sous une forme annulaire.

14. Dispositif selon l'une des revendications précédentes, **caractérisé en ce que** le calculateur (22) est programmé pour commander les moyens (24) de telle sorte qu'ils forment dans le tissu oculaire (12) des canaux (18a, 18b, 18c) qui s'étendent à des profondeurs différentes dans le tissu oculaire.
